# EUROPÄISCHE PATENTSCHRIFT

(11) **EP 0 974 371 B1**
(45) Veröffentlichungstag und Bekanntmachung des Hinweises auf die Patenterteilung: **14.06.2006**
(21) Anmeldenummer: 99113818.1
(22) Anmeldetag: 15.07.1999
(51) Int. Cl.: A61M 1/34, A61M 1/16

(54) **Verfahren und Vorrichtung zum Überprüfen des ordnungsgemässen Austausches eines gebrauchten Filters in einer Vorrichtung zur extrakorporalen Blutbehandlung**
Method and device for controlling the proper replacement of a used filter in an extracorporal blood treatment device
Dispositif et méthode pour contrôler le remplacement correct d'un filtre usé dans un appareil de traitement extracorporel du sang

(30) Priorität: 18.07.1998 DE 19832451
(43) Veröffentlichungstag der Anmeldung: 26.01.2000
(73) Patentinhaber: Fresenius Medical Care Deutschland GmbH, 61352 Bad Homburg v.d.H. (DE)
(72) Erfinder: Brauer, Helge, 97469 Gochsheim (DE)
(74) Vertreter: Luderschmidt, Schüler & Partner GbR

(56) Entgegenhaltungen:
- EP-A- 0 763 367
- DE-A- 3 444 671
- DE-C- 3 923 078

## Beschreibung

Die Erfindung betrifft ein Verfahren zum Überprüfen des ordnungsgemäßen Austausches eines gebrauchten Filters, der durch eine Membran in zwei Kammern geteilt ist, die in dem Flüssigkeitssystem einer Vorrichtung zur extrakorporalen Blutbehandlung angeordnet sind. Darüber hinaus bezieht sich die Erfindung auf eine Vorrichtung zur extrakorporalen Blutbehandlung mit einem Flüssigkeitssystem, das mindestens einen durch eine Membran in eine erste und zweite Kammer geteilten Filter enthält, wobei der ordnungsgemäße Filterwechsel überprüfbar ist.

Zur Entfernung von harnpflichtigen Substanzen und zum Flüssigkeitsentzug werden beim chronischen Nierenversagen verschiedene Verfahren zur extrakorporalen Blutbehandlung bzw. -reinigung eingesetzt. Bei der Hämodialyse wird das Blut des Patienten außerhalb des Körpers in einer künstlichen Niere gereinigt, die auch als Dialysator bezeichnet wird. Der Dialysator weist eine Blutkammer und eine Dialysierflüssigkeitskammer auf, die von einer semipermeablen Membran getrennt werden. Während der Behandlung fließt das Blut des Patienten durch die Blutkammer auf der einen Seite der Membran. Um das Blut effektiv von den harnpflichtigen Substanzen zu reinigen, wird die Dialysierflüssigkeitskammer kontinuierlich von frischer Dialysierflüssigkeit durchströmt.

Während bei der Hämodialyse (HD) der diffuse Stofftransport überwiegt, findet bei der Hämofiltration (HF) ein konvektiver Stofftransport über die Membran statt. Eine Kombination aus beiden Verfahren ist die Hämodiafiltration (HDF).

Bei der Hämo(dia)filtration wird ein Teil des durch die Membran abgezogenen Serums durch eine sterile Substitutionsflüssigkeit ersetzt, die entweder stromauf des Dialysators (Prädilution) oder stromab des Dialysators (Postdiluation) dem extrakorporalen Blutkreislauf zugesetzt wird.

Es sind Vorrichtungen zur Hämo(dia)filtration bekannt, bei denen die Dialysierflüssigkeit online aus Frischwasser und einem Elektrolytkonzentrat und die Substitutionsflüssigkeit online aus der Dialysierflüssigkeit hergestellt werden. Um sicherzustellen, dass die online hergestellte Dialysier- und Substitutionsflüssigkeit steril und pyrogenfrei sind, werden die Flüssigkeiten durch im Flüssigkeitssystem der Hämo(dia)filtrationsvorrichtung angeordnete Filter geleitet, die durch eine Keime zurückhaltende Membran in zwei Kammern geteilt sind. Eine derartige Vorrichtung mit zwei im Dialysierflüssigkeitssystem angeordneten Filtern ist aus der DE 34 44 671 C2 bekannt.

Die DE 34 48 262 C2 beschreibt ein Verfahren zum Überprüfen der Dichtheit der Filter der aus der DE 34 44 671 C2 bekannten Hämo(dia)filtrationsvorrichtung. Die Integritätsprüfung der Filter erfolgt mittels eines Druckhaltetestes, wobei in einer der beiden Kammern des Filters ein Unterdruck aufgebaut wird. Der Druckhaltetest beruht darauf, dass die mit einer Flüssigkeit benetzte Membran des Filters für Gase im Wesentlichen undurchlässig ist. Nur im Falle eines Defekts, kann während des Druckhaltetests ein Druckanstieg in der Kammer festgestellt werden.

In der DE 34 42 744 A1 wird ein Membranintegritätstest für einen gebrauchten Dialysator beschrieben, bei dem die Kammern des Dialysators mit Luft gefüllt werden und der Druckausgleich über die benetzte Membran beobachtet wird.

Aus der EP-A-0 763 367 ist ein Verfahren zum Überprüfen von im Dialysierflüssigkeitssystem einer Vorrichtung zur extrakorporalen Blutbehandlung angeordneten Filtern beschrieben. Die zu überprüfende Filtermembran wird mit einer wässrigen Flüssigkeit vollständig benetzt und ein Druckhaltetest durchgeführt. Der Druckhaltetest bildet die Grundlage für die Entscheidung, ob die Filtermembran dicht ist.

Die Hersteller der bekannten Sterilfilter für Hämo(dia)filtrationsvorrichtungen schreiben aus Sicherheitsgründen einen Filterwechsel in bestimmten Zeitabständen vor.

Es sind Hämo(dia)filtrationsvorrichtungen bekannt, die den Anwender auf den erforderlichen Filterwechsel automatisch hinweisen. Zur Erhöhung der Sicherheit sehen die bekannten Hämo(dia)filtrationsvorrichtungen auch eine manuelle Bestätigung des Filterwechsels durch den Anwender vor. Diese Sicherheuseinrichtung kann aber nicht ausschließen, daß der Anwender den Wechsel des Filters zwar bestätigt, einen Füterweehsel aber dennoch nicht durchführt.

Der Erfindung liegt die Aufgabe zugrunde, ein Verfahren anzugeben, daß es erlaubt, mit hoher Sicherheit festzustellen, ob ein gebrauchter Filter gegen einen neuen Filter ausgetauscht worden ist. Darüber hinaus liegt der Erfindung die Aufgabe zugrunde, eine Vorrichtung zur extrakorporalen Blutbehandlung anzugeben, die über eine Einrichtung verfügt, mit der sich ein ordnungsgemäßer Filterwechsel mit hoher Sicherheit feststellen läßt.

Die Lösung dieser Aufgaben erfolgt erfindungsgemäß mit den Merkmalen der Patentansprüche 1 und 6.

Bei der Erfindung wird davon ausgegangen, daß die Membran eines neuen Filters trocken und die eines gebrauchten Filters mit Flüssigkeit benetzt ist. Zum Überprüfen des ordnungsgemäßen Filterwechsels wird ein Druckhaltetest durchgeführt. Dabei wird geprüft, ob die Membran des Filters für Gas durchlässig ist. Für den Fall, daß die Membran für Gas undurchlässig ist, wird darauf geschlossen, daß die Membran mit Flüssigkeit benetzt und der Filter gebraucht ist.

Mit dem Druckhaltetest kann der ordnungsgemäße Wechsel sämtlicher im Flüssigkeitssystem der Vorrichtung zur extrakorporalen Blutbehandlung angeordneter Filter geprüft werden. Hierzu zählen sowohl die Filter zur Bereitstellung steriler Dialysier- und Substitutionsflüssigkeiten als auch der Dialysator.

Die Gasdurchlässigkeit der Membran des Filters wird durch einen Überdruck- oder Unterdrucktest geprüft. Vorzugsweise wird ein die erste Kammer des Filters einschließender Abschnitt und ein die zweite Kammer des Filters einschließender Abschnitt des Flüssigkeitssystems abgesperrt und in einen der beiden Abschnitte zum Aufbau eines Überdrucks Gas geleitet, wobei der Druck in diesem Abschnitt überwacht wird. Daraufhin wird der andere Abschnitt des Flüssigkeitssystems wieder geöffnet und bei einem Druckabfall in dem abgesperrten Abschnitt wird darauf geschlossen, dass der Filter ordnungsgemäß gewechselt worden ist. Es ist aber auch möglich, zu prüfen, ob sich in einen abgesperrten Abschnitt des Flüssigkeitssystems, der eine der beiden Kammern des Filters einschließt, ein Überdruck aufbauen lässt. Wenn dies der Fall ist, handelt es sich um einen gebrauchten Filter, dessen Membran mit Flüssigkeit benetzt ist.

Zweckmäßigerweise wird der Druckabfall pro Zeiteinheit mit einem vorgegebenen Grenzwert verglichen, so dass bei Überschreiten des Grenzwertes auf den ordnungsgemäßen Filterwechsel geschlossen werden kann. Dadurch ist sichergestellt, dass zwischen einem Druckabfall, der darauf zurückzuführen ist, dass die Membran des Filters nicht mit Flüssigkeit benetzt ist und dem nur sehr geringen Druckabfall unterschieden werden kann, der auch bei einer mit Flüssigkeit benetzten Membran über einen längeren Zeitraum festzustellen ist.

Zum Aufbau eines Überdruck wird zweckmäßigerweise Luft aus der Atmosphäre in einen der beiden Abschnitte des Flüssigkeitssystems geleitet, wobei der Überdruck mit einer Luftpumpe aufgebaut werden kann. Um zu verhindern, daß Keime in das Flüssigkeitssystem gelangen, wird die Luft vorzugsweise durch einen hydrophoben Filter geleitet.

Für den Fall, daß der gebrauchte Filter nicht gegen einen neuen Filter ausgetauscht worden ist, wird vorteilhafterweise ein optischer und/oder akustischer Alarm gegeben. Es ist aber auch möglich, in diesem Fall den weiteren Betrieb der extrakorporalen Blutbehandlungsvorrichtung zu verhindern, so daß die Behandlung mit dem verbrauchten Filter nicht mehr fortgesetzt werden kann.

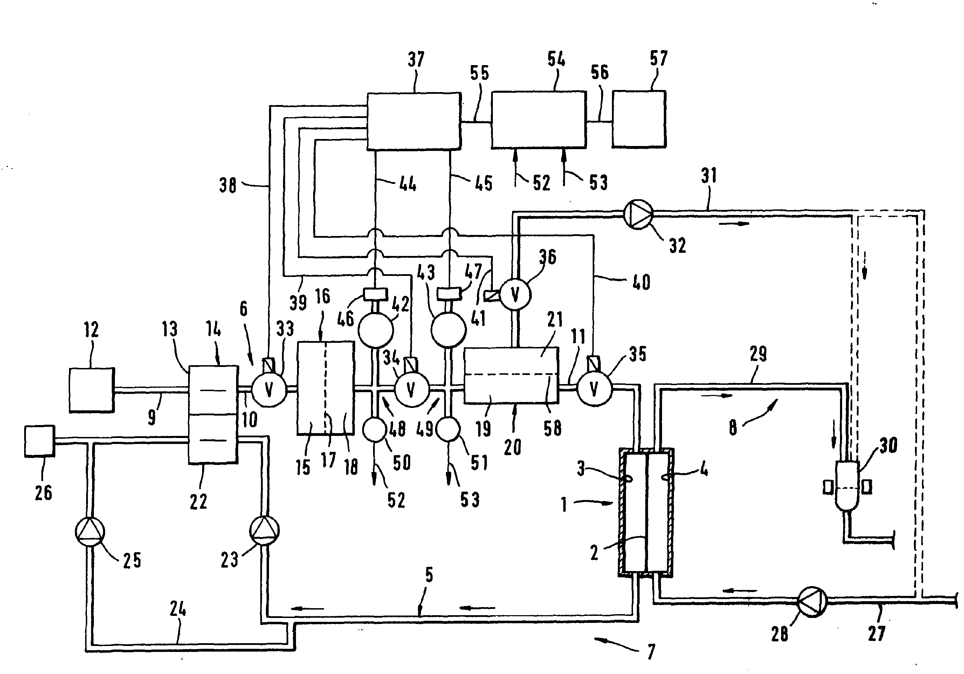

Im folgenden wird ein Ausführungsbeispiel der Erfindung unter Bezugnahme auf die Zeichnung näher erläutert, die eine Hämo(dia)filtrationsvorrichtung in schematischer Darstellung zeigt, deren Dialysierflüssigkeitssystem zwei Filter enthält.

Die Hämo(dia)filtrationsvorrichtung weist einen Dialysator 1 auf, der durch eine Membran 2 in eine von Dialysierflüssigkeit durchflossene erste Kammer 3 und eine von Blut durchflossene zweite Kammer 4 getrennt ist.

Die erste Kammer 3 ist in einen Dialysierflüssigkeitsweg 5 des Dialysierflüssigkeitssystems der extrakorporalen Blutbehandlungsvorrichtung geschaltet, der eine Zuleitung 6 und eine Ableitung 7 aufweist, während die zweite Kammer 4 in einen Blutweg 8 geschaltet ist.

Die Zuleitung 6 des Dialysierflüssigkeitsweges 5 besteht aus einem ersten Zuleitungsabschnitt 9, einem zweiten Zuleitungsabschnitt 10 sowie einem dritten Zuleitungsabschnitt 11 und verbindet eine Dialysierflüssigkeitsquelle 12 mit der ersten Kammer 3 des Dialysators 1.

Der erste Zuleitungsabschnitt 9 führt von der Dialysierflüssigkeitsquelle 12 zu der ersten Bilanzkammer 13 einer zwei Bilanzkammern aufweisenden Bilanziervorrichtung 14. Die erste Bilanzkammer 13 der Bilanziervorrichtung 14 ist über den zweiten Zuleitungsabschnitt 10 mit dem Eingang der ersten Kammer 15 eines ersten Sterilfilters 16 verbunden, der durch eine Keime zurückhaltende, hydrophile Membran 17 in eine erste und zweite Kammer 15, 18 geteilt ist. In den dritten Zuleitungsabschnitt 11 ist die erste Kammer 19 eines zweiten Sterilfilters 20 geschaltet, der ebenfalls durch eine Keime zurückhaltende, hydrophile Membran 58 in die erste und eine zweite Kammer 19,21 geteilt ist.

Vom Ausgang der ersten Kammer 3 des Dialysators 1 führt die Ableitung 7 zur zweiten Bilanzkammer 22 der Bilanziervorrichtung 14, wobei in die Ableitung eine Dialysierflüssigkeitspumpe 23 geschaltet ist. Stromaufwärts der Dialysierflüssigkeitspumpe 23 geht von der Ableitung 7 eine Ultrafiltratleitung 24 ab, in die eine Ultrafiltrationspumpe 25 zum Abziehen von Dialysierflüssigkeit geschaltet ist. Die Ultrafiltratleitung 24 führt zu einem Abfluß 26, an den auch der Ausgang der zweiten Bilanzkammer 22 der Bilanziervorrichtung 14 angeschlossen ist.

Der Blutweg 8 weist eine vom Patienten kommende Blutzuleitung 27 auf, die an den Eingang der zweiten Kammer 4 des Dialysators 1 angeschlossen ist. In die Blutzuleitung 27 ist eine Pumpe 28 geschaltet. Der Ausgang der zweiten Kammer 4 des Dialysators 1 führt über den ersten Abschnitt einer Blutableitung 29 zu einer Tropfkammer 30, von der Blut über den zweiten Abschnitt der Blutableitung 29 zum Patienten geführt wird.

Das Dialysierflüssigkeitssystem weist ferner eine Substituatleitung 31 auf, die von der zweiten Kammer 21 des zweiten Sterilfilters 20 abzweigt und wahlweise mit der Blutzuleitung 27 (Prädilution) oder mit der Tropfkammer 30 (Postdilution) verbunden werden kann. Die beiden Verbindungszweige sind durch gestrichelte Linien angedeutet. In die Substituatleitung 31 ist eine Substituatpumpe 32 geschaltet.

Die Hämo(dia)filtrationsvorrichtung verfügt über eine Einrichtung zum Überprüfen des ordnungsgemäßen Austausches der beiden Sterilfilter 16, 20. Die Einrichtung zum Überprüfen des ordnungsgemäßen Filterwechsels umfäßt ein in den zweiten Zuleitungsabschnitt 10 geschaltetes erstes Absperrorgan 33, ein in den dritten Zuleitungsabschnitt 11 stromauf des zweiten Sterilfilters 20 geschaltetes zweites Absperrorgan 34, ein in den dritten Zuleitungsabschnitt 11 stromab des zweiten Sterilfilters 20 geschaltetes drittes Absperrorgan 35 und ein viertes Absperrorgan 36, das in die Substituatleitung 31 geschaltet ist. Bei den Absperrorganen handelt es sich um elektromagnetisch betätigbare Ventile, die von einer zentralen Steuereinrichtung 37 über Steuerleitungen 38,39,40,41 angesteuert werden. An den dritten Zuleitungsabschnitt 11 ist stromauf und stromab des zweiten Absperrorgans 34 jeweils eine elektrische Luftpumpe 42,43 angeschlossen, die über Steuerleitungen 44,45 mit der zentralen Steuereinheit 37 verbunden sind. Die Luftpumpen 42,43 pumpen aus der Atmosphäre über jeweils einen hydrophoben Sterilfilter 46,47 Luft zum Aufbau eines Überdrucks in die absperrbaren Leitungsabschnitte 48,49 des Dialysierflüssigkeitssystems an. Darüber hinaus ist an den dritten Zuleitungsabschnitt 11 stromauf und stromab des zweiten Absperrorgans 34 jeweils ein Druckmesser 50,51 angeschlossen, mit denen der Druck in den abgesperrten Leitungsabschnitten überwacht werden kann.

Die Druckmesser 50,51 sind über Steuerleitungen 52,53 mit einerÜberwachungs - einrichtung 54 verbunden, die über eine Datenleitung 55 wiederum mit der zentralen Steuereinheit 37 in Verbindung steht. An der Überwachungseinrichtung 54 ist über eine Signalleitung 56 eine akustische und/oder optische Alarmeinrichtung 57 angeschlossen.

Nachfolgend wird das Verfahren zum Überprüfen des ordnungsgemäßen Filterwechsels im einzelnen beschrieben.

Zunächst schließt die zentrale Steuereinrichtung 37 die Absperrogane 33 bis 36. Anschließend setzt die Steuereinrichtung 37 die Luftpumpen 42,43 in Betrieb, so daß in den abgesperrten Leitungsabschnitten 48,49 des Flüssigkeitssystems der Hämo(dia)filtrationsvorrichtung ein Überdruck aufgebaut wird, der mit den Druckmessern 50,51 überwacht wird. Nachdem sich in den abgetrennten Leitungsabschnitten ein vorbestimmter Überdruck aufgebaut hat, schaltet die Steuereinrichtung die Luftpumpen ab und öffnet die Absperrorgane 33 und 36. Die Überwachungseinrichtung 54 vergleicht den mit den Druckmessern 50,51 gemessenen Druck mit einem vorgegebenen Grenzwert. Wenn der Druck in den die zweite Kammer 18 des ersten Sterilfilters 16 und die erste Kammer 19 des zweiten Sterilfilters 20 einschließenden Leitungsabschnitten des Flüssigkeitssystems nicht unter einen vorgegebenen Grenzwert innerhalb einer vorbestimmten Zeitdauer abfällt, steuert die Überwachungseinrichtung 54 die Alarmeinrichtung 57 zur Abgabe eines akustischen und/oder optischen Alarms an. Die Meßdauer beträgt etwa 10 Sekunden, wobei der vorgegebene Grenzwert etwa der Hälfte des Drucks entspricht, der in den abgesperrten Leitungszweigen aufgebaut wird.

Bei dem obigen Ausführungsbeispiel können anstelle von zwei Luftpumpen und zwei Druckmessern zur unabhängigen Überprüfung der beiden Sterilfilter auch eine gemeinsame Luftpumpe und ein gemeinsamer Druckmesser für die beiden abgetrennten Leitungsabschnitte vorgesehen sein.

## Patentansprüche

1. Verfahren zum Überprüfen des ordnungsgemäßen Austausches eines gebrauchten, mit Flüssigkeit benetzten Filters, der durch eine Membran in zwei Kammern geteilt ist, die in dem Flüssigkeitssystem einer Vorrichtung zur extrakorporalen Blutbehandlung angeordnet sind, **dadurch gekennzeichnet, dass** ein Druckhaltetest zum Prüfen der Gasdurchlässigkeit der Membran des zu prüfenden Filters durchgeführt wird, wobei ein Überdruck oder Unterdruck aufgebaut und der Druck überwacht wird, und dass für den Fall, dass der Druckabfall bzw. der Druckanstieg pro Zeiteinheit einen vorgegebenen Grenzwert überschreitet, darauf geschlossen wird, dass der gebrauchte, benetzte Filter gegen einen neuen, trockenen Filter ausgetauscht worden ist.

2. Verfahren nach Anspruch 1, **dadurch gekennzeichnet, dass**
ein die erste Kammer des Filters einschließender Abschnitt und ein die zweite Kammer des Filters einschließender Abschnitt des Flüssigkeitssystems abgesperrt wird,
in einem der beiden Abschnitte zum Aufbau eines Überdrucks Gas geleitet und der Druck in diesem Abschnitt überwacht wird,
der andere Abschnitt des Flüssigkeitssystems wieder geöffnet wird und
bei einem Druckabfall in dem abgesperrten Abschnitt darauf geschlossen wird, dass der gebrauchte, benetzte Filter gegen einen neuen, trockenen Filter ausgetauscht worden ist.

3. Verfahren nach Anspruch 2, **dadurch gekennzeichnet, dass** als Gas Luft aus der Atmosphäre in einen der beiden Abschnitte des Flüssigkeitssystems geleitet wird.

4. Verfahren nach Anspruch 3, **dadurch gekennzeichnet, dass** die Luft über einen hydrophoben Filter in den Abschnitt des Flüssigkeitssystems geleitet wird.

5. Verfahren nach einem der Ansprüche 1 bis 4, **dadurch gekennzeichnet, dass** für den Fall, dass der gebrauchte Filter nicht gegen einen neuen Filter ausgetauscht worden ist, ein optischer und/oder akustischer Alarm gegeben wird.

6. Vorrichtung zur extrakorporalen Blutbehandlung mit einem Flüssigkeitssystem, das mindestens einen durch eine Membran (17) in eine erste und zweite Kammer (15, 18) geteilten Filter (16) enthält, **dadurch gekennzeichnet, dass** eine Einrichtung zum Überprüfen des ordnungsgemäßen Austausches des gebrauchten, mit Flüssigkeit benetzten Filters (16) gegen einen neuen, trockenen Filter (16) vorgesehen ist, die zur Durchführung eines Druckhaltetests zum Prüfen der Gasdurchlässigkeit der Membran des zu prüfenden Filters (16) Mittel (33, 34, 42) zum Aufbau eines Überdrucks oder Unterdrucks und Mittel (50, 37, 54) zum Überwachen des Drucks aufweist, wobei die Einrichtung zum Überprüfen des ordnungsgemäßen Austausches des Filters derart ausgebildet ist, dass für den Fall, dass der Druckabfall bzw. Druckanstieg pro Zeiteinheit einen vorgegebenen Grenzwert überschreitet, durch die Mittel (50, 37, 54) zum Überwachen des Drucks festgestellt wird, dass der gebrauchte, benetzte Filter gegen einen neuen, trockenen Filter ausgetauscht worden ist.

7. Vorrichtung nach Anspruch 6, **dadurch gekennzeichnet, dass** die Mittel zum Aufbau eines Überdrucks Mittel (33) zum Absperren eines die erste Kammer (15) des Filters (16) einschließenden Abschnitts und Mittel (34) zum Absperren eines die zweite Kammer (18) des Filters einschließenden Abschnitts des Flüssigkeitssystems und Mittel (42) zum Zuführen von Gas in einen der beiden Abschnitte zum Aufbau eines Überdrucks aufweist, wobei die Mittel zum Überwachen des Drucks als Mittel zum Überwachen des Überdrucks in diesem Abschnitt des Flüssigkeitssystems ausgebildet sind.

8. Vorrichtung nach Anspruch 6 oder 7, **dadurch gekennzeichnet, dass** die Mittel zum Zuführen von Gas eine Luft aus der Atmosphäre ansaugende Luftpumpe (42) sind.

9. Vorrichtung nach Anspruch 8, **dadurch gekennzeichnet, dass** die Luftpumpe (42) einen hydrophoben Filter (46) aufweist, über den Luft aus der Atmosphäre angesaugt wird.

10. Vorrichtung nach einem der Ansprüche 6 bis 9, **dadurch gekennzeichnet, dass** die Einrichtung zum Überprüfen des ordnungsgemäßen Austausches des Filters Mittel (57) zur Abgabe eines optischen und/oder akustischen Alarms für den Fall aufweist, dass der gebrauchte, benetzte Filter nicht gegen einen neuen, trockenen Filter ausgetauscht worden ist.

## Claims

1. A method for checking the proper replacement of a used filter wetted with liquid, which is divided by a membrane into two chambers which are arranged in the liquid system of an apparatus for extracorporeal blood treatment, **characterised in that** a pressure-holding test is carried out in order to test the gas permeability of the membrane of the filter to be tested, whereby an over-pressure or under-pressure is built up and the pressure is monitored, and that, in the event of a pressure drop or a pressure rise per unit of time exceeding a predetermined limiting value, it is concluded that the used wetted filter has been replaced with a new, dry filter.

2. The method according to claim 1, **characterised in that**
a section of the liquid system including the first chamber of the filter and a section of the liquid system including the second chamber of the filter is shut off,
gas is conveyed in one of the two sections in order to build up an over-pressure and the pressure in this section is monitored,
the other section of the liquid system is opened again and,
in the event of a pressure drop in the shut-off section, it is concluded that the used, wetted filter has been replaced with a new, dry filter.

3. The method according to claim 2, **characterised in that** air from the atmosphere is conveyed as a gas into one of the two sections of the liquid system.

4. The method according to claim 3, **characterised in that** the air is conveyed via a hydrophobic filter into the section of the liquid system.

5. The method according to any one of claims 1 to 4, **characterised in that**, in the event that the used filter has not been replaced with a new filter, an optical and/or acoustic alarm is emitted.

6. An apparatus for extracorporeal blood treatment with a liquid system, which contains at least one filter (16) divided by a membrane (17) into a first and second chamber (15, 18), **characterised in that** a device is provided for checking the proper replacement of the used filter (16) wetted with liquid with a new, dry filter (16), which device has means (33, 34, 42) for building up an over-pressure or under-pressure and means (50, 37, 54) for monitoring the pressure for the purpose of performing a pressure-holding test in order to check the gas permeability of the membrane of the filter (16) to be tested, whereby the device for checking the proper replacement of the filter is designed in such a way that, in the event that the pressure drop or pressure rise per unit of time exceeds a predetermined limiting value, it is ascertained by the means (50, 37, 54) for monitoring the pressure that the used, wetted filter has been replaced with a new, dry filter.

7. The device according to claim 6, **characterised in that** the means for building up an over-pressure have means (33) for shutting off a section of the liquid system including the first chamber (15) of the filter (16) and means (34) for shutting off a section of the liquid system including the second chamber (18) of the filter and means (42) for the feeding of gas into one of the two sections in order to build up an over-pressure, whereby the means for monitoring the pressure are designed as means for monitoring the over-pressure in this section of the liquid system.

8. The device according to claim 6 or 7, **characterised in that** the means for the feeding of gas are an air pump (42) sucking air out of the atmosphere.

9. The device according to claim 8, **characterised in that** the air pump (42) has a hydrophobic filter (46), via which air is sucked from the atmosphere.

10. The device according to any one of claims 6 to 9, **characterised in that** the device for checking the proper replacement of the filter has means (57) for emitting an optical and/or acoustic alarm in the event that the used, wetted filter has not been replaced with a new, dry filter.

## Revendications

1. Procédé de contrôle du remplacement convenable d'un filtre usagé, mouillé par un liquide, qui est divisé par une membrane en deux chambres qui sont disposées dans le système de liquide d'un dispositif destiné au traitement extracorporel du sang, **caractérisé en ce qu'**un essai de maintien de la pression est réalisé pour contrôler la perméabilité au gaz de la membrane du filtre destiné à être contrôlé, une surpression ou une dépression étant établie et la pression étant contrôlée, et **en ce que** pour le cas où la chute de pression et/ou l'augmentation de pression par unité de temps dépasse une valeur seuil prédéterminée, on en conclut que le filtre usagé et mouillé a été remplacé par un nouveau filtre sec.

2. Procédé selon la revendication 1, **caractérisé en ce que**
une section enfermant la première chambre du filtre et une section enfermant la seconde chambre du filtre du système de liquide sont fermées,
le gaz est acheminé dans une des deux sections pour établir une surpression et la pression dans cette section est surveillée,
l'autre section du système de liquide est à nouveau ouverte et
à l'occasion d'une chute de pression dans la section fermée, on en conclut que le filtre usagé et mouillé a été remplacé par un nouveau filtre sec.

3. Procédé selon la revendication 2, **caractérisé en ce que** l'air est acheminé en tant que gaz depuis l'atmosphère dans une des deux sections du système de liquide.

4. Procédé selon la revendication 3, **caractérisé en ce que** l'air est acheminé par un filtre hydrophobe dans la section du système de liquide.

5. Procédé selon l'une quelconque des revendications 1 à 4, **caractérisé en ce que** pour le cas où le filtre usagé n'a pas été remplacé par un nouveau filtre, une alarme optique et/ou acoustique est déclenchée.

6. Dispositif de traitement extracorporel du sang doté d'un système de liquide qui contient au moins un filtre (16) divisé par une membrane (17) en une première et une seconde chambres (15, 18), **caractérisé en ce qu'**il est prévu un système de contrôle du remplacement convenable du filtre (16) mouillé de liquide par un nouveau filtre (16) sec, qui comprend, pour réaliser un essai de maintien de pression destiné à contrôler la perméabilité au gaz de la membrane du filtre (16) destiné à être contrôlé, des moyens (33, 34, 42) destinés à établir une surpression ou une dépression et des moyens (50, 37, 54) destinés à contrôler la pression, le système de contrôle du remplacement convenable du filtre étant conçu de telle sorte que pour le cas où la chute de pression et/ou l'augmentation de pression par unité de temps dépasse une valeur seuil prédéterminée, il est constaté par les moyens (50, 37, 54) de contrôle de la pression que le filtre usagé et mouillé a été remplacé par un nouveau filtre sec.

7. Dispositif selon la revendication 6, **caractérisé en ce que** les moyens destinés à établir une surpression comprennent des moyens (33) destinés à fermer une section enfermant la première chambre (15) du filtre (16) et des moyens (34) destinés à fermer une section du système de liquide enfermant la seconde chambre (18) du filtre et des moyens (42) destinés à amener le gaz dans une des deux sections pour établir une surpression, les moyens de surveillance de la pression étant conçus comme des moyens destinés à surveiller la surpression dans cette section du système de liquide.

8. Dispositif selon la revendication 6 ou 7, **caractérisé en ce que** les moyens destinés à amener le gaz sont une pompe à air (42) aspirant l'air de l'atmosphère.

9. Dispositif selon la revendication 8, **caractérisé en ce que** la pompe à air (42) comprend un filtre hydrophobe (46) par l'intermédiaire duquel l'air est aspiré de l'atmosphère.

10. Dispositif selon l'une quelconque des revendications 6 à 9, **caractérisé en ce que** le système de contrôle du remplacement convenable du filtre comprend des moyens (57) destinés à déclencher une alarme optique et/ou acoustique pour le cas où le filtre usagé et mouillé n'a pas été remplacé par un nouveau filtre sec.
